# EUROPEAN PATENT SPECIFICATION

(11) **EP 3 380 842 B1**
(45) Date of publication and mention of the grant of the patent: **13.11.2019**
(21) Application number: 16820341.2
(22) Date of filing: 28.11.2016
(51) Int. Cl.: G01N 33/569

(54) **METHOD TO DETECT BACTERIAL ACTIVITY IN A BIOLOGICAL SAMPLE**
VERFAHREN ZUR DETEKTION VON BAKTERIELLER AKTIVITÄT IN EINER BIOLOGISCHEN PROBE
PROCÉDÉ POUR DÉTECTER UNE ACTIVITÉ BACTÉRIENNE DANS UN ÉCHANTILLON BIOLOGIQUE

(30) Priority: 27.11.2015 IT UB20155975
(43) Date of publication of application: 03.10.2018
(73) Proprietor: Alifax S.r.l., 35020 Polverara (PD) (IT)
(72) Inventor: GALIANO, Paolo, 35100 Padova (IT)
(74) Representative: Petraz, Gilberto Luigi
(86) International application number: PCT/IB2016/057162
(87) International publication number: WO 2017/090015

(56) References cited:
- WO-A1-2006/079846
- WO-A1-2014/128629
- F GARDINI ET AL: "A head space gas chromatographic approach for the monitoring of the microbial cell activity and the cell viability evaluation", JOURNAL OF MICROBIOLOGICAL METHODS, vol. 29, no. 2, 1 May 1997 (1997-05-01), pages 103-114, XP055189508, ISSN: 0167-7012, DOI: 10.1016/S0167-7012(97)00028-6

## Description

### FIELD OF THE INVENTION

The present invention concerns a method to detect activity and presence of bacterial species in biological samples, in particular, but not only, blood samples, using techniques based on micro gas chromatography in a detection unit.

The method according to the present invention can be adopted, for example, in diagnostics for humans, in the veterinary field, food and any other field, to detect the presence of bacteria in the various biological samples analyzed.

### BACKMEDIUM OF THE INVENTION

The rapid and accurate detection of bacteria and microbe activity in a biological sample is fundamental in diagnosing infectious diseases and hence in formulating the correct antibiotic therapy.

The main direct method to detect bacterial activity provides to put a biological sample in culture mediums or broths with specific nutrient elements able to increase the growth of the bacteria.

Techniques that provide to use a culture medium or broth are characterized by a waiting time that varies according to the characteristics of the culture medium or broth and the conditions that increase the bacterial growth itself.

Another technique to detect the presence of bacteria provides to analyze the gas present in the headspace of a test tube or other suitable container, such as for example those generically called vials.

By "headspace" we mean the free volume inside a sealed test tube located above the sample examined.

Currently, test tubes are used that use pastilles disposed on the bottom of the test tube, in contact with the biological sample containing the bacterial load, with added culture medium or broth; the function of the pastilles is to absorb the gases produced by the bacteria.

The patent application US-A-2010/0255529 describes a method to detect gaseous substances of a biological sample by inoculating the latter in a culture medium in a test tube.

Furthermore, this document provides a repeated measuring of carbon dioxide (CO₂) at different intervals of time to exclude possible erroneous measurements and to define whether the biological sample is positive or negative by measuring the increase in CO₂ compared with the quantity normally present in the air, as a signal that the bacteria are present in the sample and are replicating.

If the detection of CO₂ is uncertain, that is, it does not exceed a determinate threshold value of acceptability to attest the presence of bacteria, a second step of incubation is carried out, in addition to the first step, to allow the gases to again saturate the headspace.

These techniques, although an improvement on other, more traditional procedures, require detection times of the CO₂, which varies depending on the bacterial load present in the sample, that can even take several days to incubate and allow the corresponding positive detection due to the presence of bacteria.

It often happens that the detection times are not compatible with the urgency of the response.

It is known that to analyze a gas in samples of whole blood, a device can be used that captures the gas to detect the CO₂. For example, the devices commonly known in the state of the art and proposed, for example, by Biomerieux and Becton Dickenson, can be used.

Other solutions in the food sector, described for example in document by F. Gardini et al., Journal of Microbiological Methods 29 (1997), 103-114, provide to analyze the CO₂ in the headspace of a sealed test tube in which food samples are located, correlating the number of bacteria present in the latter only with the percentage of CO₂ measured in the headspace.

This solution is limited to this particular field, since it allows to obtain information on the number of bacteria in the food sample, measuring high quantities of CO₂, that is, quantities higher than 300 ppm (parts per million), which are considerably higher than the quantities of CO₂ typically present in the case of blood samples.

The solution described by F. Gardini et al. also requires long times to obtain results which do not give precise and reliable indications when the quantities of CO₂ are lower than 300 ppm.

Alternatively, the bacterial presence can be detected by detecting the variation in pressure determined and measured by a sensor located on the stopper of the test tube itself, for example using Versatrek-Thermofischer devices.

Another technique to detect bacterial activity provides to evaluate the difference in pressure inside the test tube, without identifying the gaseous species detected that are measured, for example CO₂, O₂, H₂.

Other known solutions, such as for example WO 2014/128629 (WO'629) and WO2006/079846 (WO'846), describe methods to identify bacterial species present in the sample analyzed, removing and analyzing the volatile substances, which in this specific case refer to organic substances or organically derived substances, present in the headspace.

These known solutions provide to make a single measurement, also called one spot, which does not give information concerning the growth and replication of the bacteria possibly present in the sample examined.

One purpose of the present invention is to perfect a method to detect the activity and presence of bacteria in a biological sample that is rapid, easy to apply and that guarantees sure results.

Another purpose of the present invention is to provide a method that allows to detect the presence of CO₂, and O₂ simultaneously, allowing to simultaneously verify, for example, an increase in the quantity of CO₂, and a decrease in O₂, that is used to form the Carbon and Oxygen bond. The measurement can occur in a continuous measuring flow of the gaseous components.

Another purpose is to provide a method that can reduce the quantity of biological sample needed to detect the bacteria present which, with known methods to detect CO₂, in blood samples, requires test tubes in which more than 10 ml of whole blood are used.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purposes, the present invention concerns a method to detect bacterial activity, that is, live bacteria replicating in a biological sample by analyzing inorganic gaseous substances, such as in particular but not only CO₂, H₂, O₂ in the headspace of a sealed test tube inside which the biological sample has been introduced.

The present invention is based on the principle that the live bacteria have one of their active metabolisms that entails the development of CO₂ as a sign of their metabolism and the contemporary measuring of O₂ decreasing.

The measurements of CO₂ and O₂ are advantageously carried out as a dynamic measurement, that is, in a continuous flow of the gaseous substances inside the headspace at various reading times.

The sequence of dynamic measurements can thus supply a temporal measuring curve, from which the active dynamic of the bacterial replication is constructed that, in particular, is determined by the increase in CO₂ and by the decrease in O₂ that bonds with the carbon, in the headspace of the biological sample inserted in a sealed vial.

The method is also applied in the detection of fungus and yeasts present in biological samples.

The method thus provides to remove the gases contained in the headspace in order to analyze the content of inorganic gaseous substances such as in particular CO₂, H₂ and/or O₂.

In one embodiment, in order to facilitate the replication of the bacteria possibly present in a biological sample, the invention provides to introduce, into the headspace, adjuvant substances such as hydrocarbons (methane, ethane, propane and other similar or comparable substances) that speed up bacterial replication.

In an advantageous embodiment, the present invention provides to introduce lysant substances inside the vial for collecting samples, which break up the red blood cells and allow the intercellular bacteria to replicate in the sample test tube.

The detection of the presence of the inorganic gaseous substances detected, for example, at a time T0 at start-of-reading and re-read over time at subsequent intervals T1, T2, ..., Tn, allows to construct a temporal detection curve, which, on the basis of its growth dynamic, and therefore its exponential detection, allows to reduce the detection times of the presence of bacteria induced by the detection of the inorganic substances.

Unlike applications known in the state of the art, the invention thus allows to obtain a detection growth curve and such as to quantify as far as ppm quantities.

According to possible embodiments, the invention therefore allows to identify inorganic substances correlated to the presence of bacteria in the biological sample, such as CO₂ for example, in a range comprised between 1ppm and 10 ppm.

In one embodiment, it is also provided to introduce a culture medium or broth provided with nutritive substances for the bacteria and that, advantageously, increase their growth. In this way a culture is created favorable to bacterial growth with consequent reduction in the times needed for analysis.

In one embodiment, the analysis for the detection of variations in concentration of CO₂, H₂ and/or O₂ occurs by using a high-speed, miniaturized gas chromatograph with heat conductivity detection.

In particular, by using a micro gas chromatograph, the removal is provided of a quantity of gas from the headspace by introducing a needle inside the test tube.

In this solution, the needle is connected to a suction member able to remove the gases that have developed inside the headspace of the test tube.

According to a variant of the invention, the re-introduction of the quantity of gas taken from the inside of the test tube by means of a second needle is also provided.

The re-introduction of the volume of the headspace inside the micro gas chromatograph allows to create a circulation of gas that facilitates the detection of the inorganic gaseous substances by the micro gas chromatograph. The measuring flow allows to measure the increase values (delta) of the gaseous quantities detectable over time, so as to allow the pump connected to the needle to have a gaseous substance available to be aspirated. The continuous flow measurement allows the second sampling to find gas measurable and available, and to compare in the measuring times the increase in CO₂ and the decrease in O₂.

Without this flow re-circling the second sampling would not find gas available, since it would have been extracted by the first sampling.

In one embodiment, a magnetic element can be provided inside the test tube in order to allow the stirring of the biological sample, or the bacterial culture. The mixing of the sample under examination helps the bacterial replication in order to supply nutriment material in the culture broth, as well as facilitating the lysis of the red blood cells with the insertion of the lysant substances.

### BRIEF DESCRIPTION OF THE DRAWINGS

These and other characteristics of the present invention will become apparent from the following description of some embodiments, given as a non-restrictive example with reference to the attached drawings wherein:

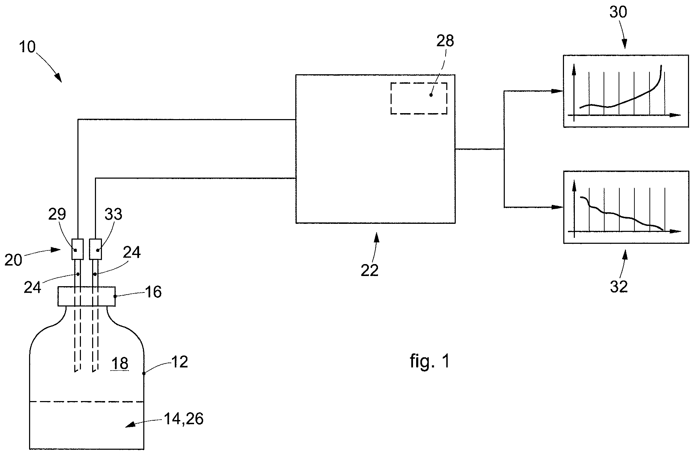

- fig. 1 is a schematic representation of a detection method according to one embodiment.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

Fig. 1 is used to describe a method 10 to detect bacterial activity, that is, to detect the existence of live and replicating bacteria, by detecting the presence and quantity of CO₂, H₂ and/or O₂ in a hermetically sealed test tube with a biological sample.

For example, biological sample 14 can comprise, but not only, blood, urine, saliva, mucus, tears or other suitable sample.

In one embodiment, the biological sample 14 is inserted in a test tube or vial 12, suitably sealed and sterilized.

The test tube 12 is sealed by a stopper 16 which comprises a membrane, for example rubber, self-sealing and which allows needles to pass for example.

The biological sample 14 is analyzed by introducing inside the test tube 12 a minimum amount of biological sample 14, for example less than 5 ml.

This aspect advantageously allows to apply the present method even when small quantities of biological sample 14 are available, for example in the case of analyses for newborns.

According to one aspect of the present invention, a free volume or headspace 18 is left inside the test tube 12, defined between the stopper 16 and the level of the biological sample 14, that is, above the biological sample 14.

The accumulation of gas or volatile substances is allowed in the headspace 18, that is, inorganic gaseous substances produced by the bacteria (bacterial catabolism) during their growth inside the test tube 12.

Here and hereafter we will refer to volatile substances understood as inorganic gaseous substances, such as CO₂, H₂ and/or O₂, which due to how they are defined in the present disclosure do not comprise organic substances.

In one embodiment, a volumetrically fixed quantity of gas is sent to a detection unit 22, described hereafter.

Therefore, at least one step is provided of taking a volatile sample from the headspace 18, present inside the test tube 12.

In another embodiment, the detection unit 22 is a micro gas chromatograph 22 to perform the GC analysis.

The micro gas chromatograph 22, which is suitably configured to be miniaturized and reduce to a minimum the transfer bulk, is also equipped with a device to perforate the stopper 16.

In one embodiment, taking the volatile sample from inside the headspace 18 provides to use a needle device 20 which perforates the stopper 16 of the test tube 12 and sucks up a desired quantity of gaseous mass above the biological sample 14 inside which there is the gas generated by the bacteria.

The volatile sample is subsequently analyzed by the micro gas chromatograph 22 to detect the presence and/or quantity of CO₂, H₂ and/or O₂.

In particular, the micro gas chromatograph 22 provides a control and command device 28.

According to possible embodiments, the micro gas chromatograph 22 is configured to identify inorganic substances, such as for example CO₂, H₂ and/or O₂, correlated to the presence of bacteria in the biological sample, in a range comprised between 1ppm and 10 ppm.

The control and command device 28 is able to process a detection pattern of CO₂ 30, that is, of the gaseous catabolic substances produced and present in the headspace 18.

Furthermore, as an alternative or in addition to measuring the CO₂, the control and command device 28 is able to process a detection pattern O₂ 32.

The possibility of measuring simultaneously the temporal variation of two or more inorganic gaseous substances such as CO₂ and/or O₂ allows to correlate the respective patterns and to obtain a precise, reliable and complete result.

Moreover, the measurement is carried out by re-introducing the gas into the headspace after every measurement, so that it is possible to carry out measurements in sequence and obtain the temporal evolution of each substance and hence of the correlated bacterial activity.

In particular, a removal and subsequent re-introduction procedure can provide that, in a measuring time 0, the whole gas component inside the test tube is removed. A second reading of the same test tube at time T1 would give a vacuum, alias a negative pressure, due to having aspirated the whole substance already before suction.

Therefore, re-introduction of the substance aspirated into the same test tube allows on the second, third or fourth reading at times T0, T1, T2, T3 ... a measurement assessment for the components object of the detection of the bacteria.

In the temporal and subdivided measurements at times T0, T1, T2, T3 ... it is therefore possible to detect whether the inorganic component increases or is stable over time. In one embodiment, the needle device 20 comprises a needle 24 to take a volatile sample from the headspace 18.

The taking of the volatile sample is made possible by the positive pressure in the test tube 12 which allows the volatile sample to enter inside the micro gas chromatograph 22.

In one embodiment, the volatile sample can be taken from the test tube 12 by means of aspiration using a suction member 29 integrated into the circuit of the needle device 20 and connected to the needle 24, to facilitate the measuring of the gaseous species inside the headspace 18, also for small concentrations of the gaseous species present and being examined, if the pressure inside the test tube 12 were equal to atmospheric or negative pressure.

In another embodiment, the needle device 20 comprises two needles 24. In particular, the second needle 24 is suitable to re-introduce the quantity of gas taken from the headspace 18 inside the test tube 12.

The second needle 24 can be connected to an introduction member 33 of the quantity of volatile sample inside the test tube 12.

The presence of two needles 24 allows to apply a recirculation of the gas inside the test tube 12, preventing waiting times, previously described in the state of the art, to release CO₂ into the headspace 18.

Furthermore, the periodic measurement of CO₂, O₂ will be increased as the metabolism of the bacteria increases, also facilitating the possible periodic measurement of O₂. The periodic measuring of the headspace, carried out at one or more defined time intervals, gives a dynamic measurement, that is, correlated to time, as a function of the quantity of bacteria present. The micro gas chromatograph 22 thus allows to detect growing quantities of gaseous substances to be detected and measured, so as to obtain a growth dynamic, possibly represented by a graph, of the gaseous substances detected with respect to time.

In one embodiment, the biological sample 14 can be introduced, inoculated, inside a test tube 12 where there is a culture broth.

The biological sample 14, together with the culture medium or broth forms a bacterial culture 26 after waiting for the incubation period.

In another embodiment, the method provides exclusively to introduce the native biological sample 14 inside the test tube 12 without eugonic broth.

In another embodiment, adjuvant substances can be introduced inside the test tube 12, which are able to accelerate the metabolic process of the bacterial species possibly present in the biological sample 14.

For example, by adjuvant substances we mean gaseous substances such as methane, ethane, propane or other gases, or liquid or solid substances.

In another embodiment, lysant substances can be introduced inside the biological sample 14 or the bacterial culture 26, so as to liberate the bacteria inside the red blood cells.

For example, by sequestrant substances we mean carbon or resins or other substances able to perform a sequestrant action on the antibiotic substances present in the sample following the start of an antibiotic therapy.

In another embodiment, not shown in the drawings, a magnetic element is introduced into the bottom of the test tube 12 to stir the bacterial culture 26.

The magnetic element interacts with a stirring device that causes it to rotate, and thus facilitates contact of the bacteria with the metabolic substances present in the culture medium or broth, increasing their growth and improving the mixing of the lysant substances in order to break the red blood cells inside which bacteria can exist.

The analysis of the gases, in particular CO₂ and/or O₂, present in the volatile sample, using a micro gas chromatograph 22, allows to obtain a result very quickly, for example from 5 to 40 seconds.

In one embodiment, the method can also be applied with particular types of vacuum test tubes 12.

In this way it is possible to take the biological sample 14 directly from a system to remove biological liquids, for example blood, directly from the patient.

According to a variant embodiment, if it became necessary, the method 10 can provide a step of detecting the pressure inside the test tube 12. In this way it is possible to detect an additional parameter to identify the class or species of bacteria present in the biological sample.

For example, it is possible to measure and detect the gaseous species present in the headspace 18, produced by the bacterial catabolism, and at the same time to measure the total variation in pressure in the test tube 12 by means of a miniaturized sensor.

It is clear that modifications and/or additions of parts may be made to the method 10 and detection unit 22 as described heretofore, without departing from the field and scope of the present invention. It is also clear that, although the present invention has been described with reference to some specific examples, a person of skill in the art shall certainly be able to achieve many other equivalent forms of method 10 and detection unit 22, having the characteristics as set forth in the claims and hence all coming within the field of protection defined thereby.

## Claims

1. Method to detect bacterial activity in a biological sample (14), in particular, but not only, blood samples, **characterized in that** it provides to:
- introduce the biological sample (14) into a sealed and sterilized test tube (12);
- define a headspace (18) for the accumulation of gas inside said test tube (12) and above the biological sample (14);
- take a volatile sample from said headspace;
- analyze the content of the inorganic gaseous substances, such as in particular CO₂, H₂ and/or O₂ present in said volatile sample by means of a micro gas chromatograph (22) with a flow suitable to detect the presence of inorganic substances generated by the bacterial metabolism in said biological sample (14) in a range comprised between 1 ppm and 10 ppm, said detection of the inorganic substances being carried out in a continuous flow at various sampling times to obtain a growth curve relating to the inorganic substances measured both with increasing CO₂ and decreasing O₂.

2. Method as in claim 1, **characterized in that** it provides to take a volatile sample from the headspace (18) and at the same time to measure the total variation in pressure in the test tube (12) by means of a sensor.

3. Method as in any claim hereinbefore, **characterized in that** it provides to:
- take a volatile sample from said headspace (18) by introducing a needle (24) into the test tube (12) through a stopper (16);
- re-introduce the quantity of volatile sample taken from the test tube (12) into the test tube (12) using a second needle (24) connected to an introduction member (33) in order to allow the re-circulation of the gaseous volume present in the headspace (18).

4. Method as in any claim hereinbefore, **characterized in that** it provides to carry out, by means of said micro gas chromatograph (22), a dynamic measurement of the gaseous substances present in said headspace (18), in one or more determinate time intervals, in order to obtain a growth dynamic of the individual inorganic gaseous substances present in said headspace (18).

5. Method as in claim 3 or 4, **characterized in that**, after every taking of the volatile sample, it provides to re-introduce the volatile sample in said headspace, and to perform a new measuring in sequence, in order to detect if the organic component inside it increases or is constant over time.

6. Method as in any claim hereinbefore, **characterized in that** it provides to introduce a culture medium or broth together with the biological sample (14) inside the test tube (12).

7. Method as in any claim hereinbefore from 1 to 5, **characterized in that** it provides the exclusive introduction of the native biological sample (14) inside the test tube (12).

8. Method as in any claim hereinbefore, **characterized in that** it provides to introduce adjuvant substances into said test tube (12), suitable to speed up the bacterial replication.

9. Method as in claim 8, **characterized in that** said adjuvant substances are hydrocarbons, such as methane, ethane, propane or similar or comparable substances.

10. Method as in claim 8, **characterized in that** it provides to use lysing substances able to increase the presence and/or the detection capacity of the bacteria in said biological sample (14), said lysing substances being able to increase the detection of bacteria inside the red blood cells.

11. Method as in any claim hereinbefore, **characterized in that** it provides to introduce a magnetic element to stir the biological sample (14).

12. Method as in any claim hereinbefore, **characterized in that** it provides to introduce said biological sample (14) into a test tube (12) under vacuum, to take the biological sample (14) directly from a patient.

13. Method as in any claim hereinbefore, **characterized in that** sequestrant substances of possible antibiotic substances are introduced inside a biological sample (14) or a bacterial culture (26).

14. Method as in any claim hereinbefore, **characterized in that** it provides to detect the pressure present in the test tube (12).

## Patentansprüche

1. Verfahren zum Erfassen von bakterieller Aktivität in einer biologischen Probe (14), insbesondere, aber nicht nur, Blutproben, **dadurch gekennzeichnet, dass** es bereitstellt:
- Einführen der biologischen Probe (14) in ein versiegeltes und sterilisiertes Teströhrchen (12),
- Definieren eines Gasraums (18) für die Ansammlung von Gas im Inneren des besagten Teströhrchens (12) und über der biologischen Probe (14),
- Entnehmen einer flüchtigen Probe aus dem besagten Gasraum,
- Analysieren des Gehalts der anorganischen gasförmigen Substanzen, wie insbesondere CO₂, H₂ und/oder O₂, welche sich in besagter flüchtiger Probe befinden, mittels eines Mikro-Gas-Chromatographen (22) mit einem Strom, geeignet um die Anwesenheit von anorganischen Substanzen, welche durch den bakteriellen Metabolismus in besagter biologischer Probe (14) in einem Bereich von 1 ppm bis 10 ppm erzeugt werden, zu erfassen, wobei besagtes Erfassen der anorganischen Substanzen durchgeführt wird in einem kontinuierlichen Strom zu verschiedenen Probezeiten, um eine Wachstumskurve zu erhalten, welche sich auf die anorganischen Substanzen bezieht, gemessen mit jeweils zunehmendem CO₂ und abnehmendem O₂.

2. Verfahren gemäß Anspruch 1, **dadurch gekennzeichnet, dass** es bereitstellt ein Entnehmen einer flüchtigen Probe aus dem Gasraum (18) und gleichzeitig ein Messen der gesamten Schwankung des Drucks im Teströhrchen (12) mittels eines Sensors.

3. Verfahren gemäß irgendeinem vorhergehenden Anspruch, **dadurch gekennzeichnet, dass** es bereitstellt:
- Entnehmen einer flüchtigen Probe aus besagtem Gasraum (18) durch Einführen einer Nadel (24) in das Teströhrchen (12) durch einen Verschluss (16),
- Wieder-Einführen der Menge an flüchtiger Probe, die aus dem Teströhrchen (12) entnommen wurde, in das Teströhrchen (12) durch Benutzen einer zweiten Nadel (24), welche verbunden ist mit einem Einführungselement (33), um die Rückführung des Gasvolumens, welches sich im Gasraum (18) befindet, zu ermöglichen.

4. Verfahren gemäß irgendeinem vorhergehenden Anspruch, **dadurch gekennzeichnet, dass** es bereitstellt ein Durchführen, mittels des besagten Mikro-Gas-Chromatographen (22), einer dynamischen Messung der gasförmigen Substanzen, welche sich in besagtem Gasraum (18) befinden, in einem oder mehreren bestimmten Zeitintervallen, um eine Wachstumsdynamik der individuellen, anorganischen, gasförmigen Substanzen, welche sich in besagtem Gasraum (18) befinden, zu erhalten.

5. Verfahren gemäß Anspruch 3 oder 4, **dadurch gekennzeichnet, dass** es nach jeder Entnahme der flüchtigen Probe bereitstellt ein Wieder-Einführen der flüchtigen Probe in besagten Gasraum und ein Durchführen einer neuen Messung in Sequenz, um zu erfassen, ob der organische Bestandteil im Inneren im Laufe der Zeit ansteigt oder konstant bleibt.

6. Verfahren gemäß irgendeinem vorhergehenden Anspruch, **dadurch gekennzeichnet, dass** es bereitstellt ein Einführen eines Kulturmediums oder einer Kulturbrühe zusammen mit der biologischen Probe (14) in das Teströhrchen (12).

7. Verfahren gemäß einem der Ansprüche 1 bis 5, **dadurch gekennzeichnet, dass** es die alleinige Einführung der nativen biologischen Probe (14) in das Teströhrchen (12) bereitstellt.

8. Verfahren gemäß irgendeinem vorhergehenden Anspruch, **dadurch gekennzeichnet, dass** es bereitstellt ein Einführen ergänzender Substanzen in besagtes Teströhrchen (12), geeignet um die bakterielle Replikation zu beschleunigen.

9. Verfahren gemäß Anspruch 8, **dadurch gekennzeichnet, dass** besagte ergänzende Substanzen Kohlenwasserstoffe, wie Methan, Ethan, Propan oder ähnliche oder vergleichbare Substanzen sind.

10. Verfahren gemäß Anspruch 8, **dadurch gekennzeichnet, dass** es bereitstellt ein Verwenden lysierender Substanzen, welche imstande sind die Anwesenheit und/oder die Erfassungskapazität der Bakterien in besagter biologischer Probe (14) zu erhöhen, wobei die besagten lysierende Substanzen imstande sind, die Erfassung von Bakterien in den roten Blutkörperchen zu erhöhen.

11. Verfahren gemäß irgendeinem vorhergehenden Anspruch, **dadurch gekennzeichnet, dass** es bereitstellt ein Einführen eines magnetischen Elements zum Rühren der biologischen Probe (14).

12. Verfahren gemäß irgendeinem vorhergehenden Anspruch, **dadurch gekennzeichnet, dass** es bereitstellt ein Einführen besagter biologischer Probe (14) in ein Teströhrchen (12) unter Vakuum, um die biologische Probe (14) direkt von einem Patienten zu entnehmen.

13. Verfahren gemäß irgendeinem vorhergehenden Anspruch, **dadurch gekennzeichnet, dass** komplexbildende Substanzen von möglichen antibiotischen Substanzen in eine biologische Probe (14) oder eine bakterielle Kultur (26) eingeführt werden.

14. Verfahren gemäß irgendeinem vorhergehenden Anspruch, **dadurch gekennzeichnet, dass** es bereitstellt ein Erfassen des im Teströhrchen (12) vorliegenden Drucks.

## Revendications

1. Méthode de détection d'activité bactérienne dans un échantillon biologique (14), en particulier, mais pas uniquement, des échantillons de sang, **caractérisée en ce qu'**elle comprend les étapes consistant à :
- introduire l'échantillon biologique (14) dans un tube à essai scellé et stérilisé (12) ;
- définir un espace de tête (18) pour l'accumulation de gaz à l'intérieur dudit tube à essai (12) et au-dessus de l'échantillon biologique (14) ;
- prélever un échantillon volatil à partir dudit espace de tête ;
- analyser le contenu des substances gazeuses inorganiques, comme en particulier CO₂, H₂ et/ou O₂, présentes dans ledit échantillon volatil au moyen d'un micro-chromatographe en phase gazeuse (22) avec un écoulement approprié pour détecter la présence de substances inorganiques générées par le métabolisme bactérien dans ledit échantillon biologique (14) dans une plage comprise entre 1 ppm et 10 ppm, ladite détection des substances inorganiques étant effectuée dans un écoulement continu à différentes périodes d'échantillonnage pour obtenir une courbe de croissance relative aux substances inorganiques mesurée à la fois avec augmentation du CO₂ et diminution de l'O₂.

2. Méthode selon la revendication 1, **caractérisée en ce qu'**elle comprend l'étape consistant à prélever un échantillon volatil à partir de l'espace de tête (18) et à mesurer simultanément la variation de pression totale dans le tube à essai (12) au moyen d'un capteur.

3. Méthode selon l'une quelconque des revendications précédentes, **caractérisée en ce qu'**elle comprend l'étape consistant à :
- prélever un échantillon volatil à partir dudit espace de tête (18) en introduisant une aiguille (24) dans le tube à essai (12) à travers un bouchon (16) ;
- réintroduire la quantité d'échantillon volatil prélevée à partir du tube à essai (12) dans le tube à essai (12) à l'aide d'une seconde aiguille (24) reliée à un élément d'introduction (33) afin de permettre la recirculation du volume présent dans l'espace de tête (18).

4. Méthode selon l'une quelconque des revendications précédentes, **caractérisée en ce qu'**elle comprend l'étape consistant à effectuer, au moyen dudit micro-chromatographe en phase gazeuse (22), une mesure dynamique des substances gazeuses présentes dans ledit espace de tête (18), durant un ou plusieurs intervalles de temps déterminés, afin d'obtenir une dynamique de croissance des substances gazeuses inorganiques individuelles présentes dans ledit espace de tête (18).

5. Méthode selon la revendication 3 ou 4, **caractérisée en ce que,** après chaque prélèvement de l'échantillon volatil, il est prévu de réintroduire l'échantillon volatil dans ledit espace de tête, et d'effectuer une nouvelle mesure en séquence, afin de détecter si le composant organique à l'intérieur de celui-ci augmente ou est constant dans le temps.

6. Méthode selon l'une quelconque des revendications précédentes, **caractérisée en ce qu'**elle comprend l'étape consistant à introduire un milieu ou un bouillon de culture en même temps que l'échantillon biologique (14) à l'intérieur du tube à essai (12).

7. Méthode selon l'une quelconque des revendications 1 à 5 ci-dessus, **caractérisée en ce qu'**elle comprend l'étape consistant à introduire de manière exclusive l'échantillon biologique natif (14) à l'intérieur du tube à essai (12).

8. Méthode selon l'une quelconque des revendications précédentes, **caractérisée en ce qu'**elle comprend l'étape consistant à introduire dans ledit tube à essai (12) des substances adjuvantes, adaptées pour accélérer la réplication bactérienne.

9. Méthode selon la revendication 8, **caractérisée en ce que** lesdites substances adjuvantes sont des hydrocarbures, tels que le méthane, l'éthane, le propane ou des substances similaires ou comparables.

10. Méthode selon la revendication 8, **caractérisée en ce qu'**elle comprend l'étape consistant à utiliser des substances de lyse capables d'augmenter la présence et/ou la capacité de détection des bactéries dans ledit échantillon biologique (14), lesdites substances de lyse pouvant augmenter la détection de bactéries à l'intérieur des globules rouges.

11. Méthode selon l'une quelconque des revendications précédentes, **caractérisée en ce qu'**elle comprend l'étape consistant à introduire un élément magnétique pour agiter l'échantillon biologique (14).

12. Méthode selon l'une quelconque des revendications précédentes, **caractérisée en ce qu'**elle consiste à introduire ledit échantillon biologique (14) dans un tube à essai (12) sous vide, pour prélever l'échantillon biologique (14) directement auprès d'un patient.

13. Méthode selon l'une quelconque des revendications précédentes, **caractérisée en ce que** des substances séquestrantes de substances antibiotiques possibles sont introduites à l'intérieur d'un échantillon biologique (14) ou d'une culture bactérienne (26).

14. Méthode selon l'une quelconque des revendications précédentes, **caractérisée en ce qu'**elle consiste à détecter la pression présente dans le tube à essai (12).
